(19) Europäisches Patentamt
European Patent Office
Office européen des brevets

(11) Publication number: **0 055 529**
**B2**

(12) **NEW EUROPEAN PATENT SPECIFICATION**

(45) Date of publication of the new patent specification: **19.09.90**

(21) Application number: **81305735.3**

(22) Date of filing: **04.12.81**

(51) Int. Cl.[5]: **C 01 B 33/34,** C 01 B 33/20, C 01 B 35/12, C 01 G 17/00, C 01 G 28/02, C 01 G 31/00, C 01 G 37/14, C 01 G 39/00, C 01 G 45/12, C 01 G 49/00, B 01 J 29/28 // B01J29/36, B01J31/02

# (54) Zeolites.

(30) Priority: **19.12.80 GB 8040781**

(43) Date of publication of application: **07.07.82 Bulletin 82/27**

(45) Publication of the grant of the patent: **24.04.85 Bulletin 85/17**

(45) Mention of the opposition decision: **19.09.90 Bulletin 90/38**

(84) Designated Contracting States: **DE FR GB NL**

(56) References cited:
**FR-A-2 369 871**
**US-A-4 016 245**

**INDUSTRIAL AND ENGINEERING CHEMISTRY, PROCESS DESIGN AND DEVELOPMENT, vol. 14, no. 1, January 1975 WASHINGTON (US) J.F. GIANNETTI et al.: "Selective hydrocracking with ferrieritebased catalysts" pages 86-92**

**CHEMICAL ABSTRACTS, vol. 82, no. 2, January 13, 1975, abstract no. 7895x, page 238 COLUMBUS, OHIO (US)**

(73) Proprietor: **IMPERIAL CHEMICAL INDUSTRIES PLC**
**Imperial Chemical House, Millbank**
**London SW1P 3JF (GB)**

(72) Inventor: **Seddon, Duncan**
**132 Templestowe Road Lower Templestowe**
**Melbourne Victoria 3107 (AU)**
Inventor: **Whittam, Thomas Vincent**
**30 Wilton Drive**
**Darlington Cleveland (GB)**

(74) Representative: **Chapman, Kenneth Hazel et al**
**Imperial Chemical Industries PLC Legal Department: Patents P.O. Box 6 Bessemer Road**
**Welwyn Garden City Herts, AL71HD (GB)**



Courier Press, Leamington Spa, England.

## Description

The present invention relates to a zeolite material, hereinafter referred to as zeolite FU9, and to a method of making it.

According to the present invention we provide a synthetic zeolite material, designated zeolite FU-9, as freshly made having a molar composition expressed by the formula:

$$0 \text{ to } 0.8\ M_2O : 0.1 \text{ to } 20\ Q : Y_2O_3 : 10 \text{ to } 30\ XO_2 : 0 \text{ to } 600\ H_2O$$

wherein M is an alkali metal or ammonium, Q is a tetramethylammonium ion and $M_2O+Q$ is equal to or greater than 1.0, X is silicon and/or germanium, Y is one or more of aluminum, iron, chromium, vanadium, molybdenum, arsenic, manganese, gallium or boron, and $H_2O$ is water of hydration, and having an X-ray diffraction pattern substantially as set out in Table 1 (as determined by standard technique using copper Kα radiation). Table 1 shows X-ray data for zeolite FU-9 as prepared.

| | | | | | | |
|---|---|---|---|---|---|---|
| dA | 11,3 | 9,5 | 7,05 | 6,99 | 6,61 | 5,77 |
| $100^I/Io$ | 7 | 100 | 21 | 22 | 19 | 13 |
| dA | 5,67 | 4,97 | 4,84 | 4,75 | 4,57 | 3,99 |
| $100^I/Io$ | 3 | 8 | 1 | 2 | 2 | 52 |
| dA | 3,94 | 3,85 | 3,78 | 3,66 | 3,56 | 3,53 |
| $100^I/Io$ | 37 | 18 | 32 | 14 | 40 | 55 |
| dA | 3,49 | 3,38 | 3,31 | 3,14 | 3,05 | 2,950 |
| $100^I/Io$ | 52 | 10 | 15 | 21 | 8 | 9 |
| dA | 2,898 | 2,713 | 2,643 | 2,617 | 2,575 | 2,545 |
| $100^I/Io$ | 5 | 3 | 4 | 1 | 3 | 1 |
| dA | 2,477 | 2,414 | 2,347 | 2,308 | 2,260 | 2,150 |
| $100^I/Io$ | 3 | 2 | 3 | 1 | 1 | 2 |
| dA | 2,109 | 2,027 | 1,998 | | | |
| $100^I/Io$ | 2 | 2 | 6 | | | |

"Freshly prepared" means the product of synthesis and washing, with optional drying, as hereinafter described. In freshly prepared zeolite FU9, M may include an alkali metal cation especially sodium, and/or ammonium, and Q includes nitrogen-containing organic cations as described below or cationic degradation products thereof, or precursors thereof.

Since FU9 is a zeolite, the nitrogen containing base must be physically trapped within the crystal lattice. It can be removed by thermal or oxidative degradation or by displacement by suitable small molecules. This physically trapped basic material does not constitute part of the composition for the purposes of definition.

The $H_2O$ content of freshly prepared zeolite FU9 depends on the conditions in which it has been dried after synthesis.

We believe that FU9 is a member of the ferrierite family of zeolites, which comprises, also naturally occurring ferrierite as well as zeolite SrD (J Chem Soc (1964) 485) and ZSM35 (US Patent 4,016,245). From X-ray diffraction data shown in Table 2, it seems unlikely that all of these zeolites are base on variations on the ferrierite framework which was derived by P A Vaughan (Acta Cryst (1966) *21* 983). The best fit to the calculated data from the Vaughan structure is given by zeolite FU9, virtually all the predicted lines are present in FU9. Also FU9 data gives an excellent fit to the predicted orthorhombic unit cell.

Further confirmation of these differences is provided by electron microscopy and electron diffraction studies. Under the electron microscope, ferrierite is shown to consist of irregular pieces of various sizes ranging from less than 1 micron to over 4 microns in length, ZSM35 consists of very thin rods and FU9 of regular small crystals. Natural ferrierite and ZSM35 are shown to be closely related having a similar faulted structure with fault lines perpendicular to the b axis. Zeolite FU9 appears as an idealised structure containing no apparent faults.

Still further confirmation of these differences comes from results obtained from sorption and catalytic studies, and even from the preparation and compositional properties. Comparisons of sorptive behaviour are given in Tables 3 and 4.

The Vaughan structure for ferrierite has 10 ring tunnels running down the c axis, and 8 ring tunnels along the b axis. The voidage available is approximately 16 cc per g of zeolite. From Tables 3 and 4 it can be seen that there are significant differences in sorptive behaviour between the ferrierite family members. From Table 4 it is clear that voidages in the various ferrierites are available to markedly different extents for molecules larger than methanol, water voidages are similar, and suggest part of the framework is hydrophobic, since the level is only 60—70% of that for methanol. However, voidages available to cyclic compounds differ substantially and for FU9 it seems possible that only the 10 ring tunnel system is available for n-hexane sorption whereas all the voidage appears to be available in the other forms of ferrierite. With reference to sorption of xylene isomers, FU9 is nearer to zeolite SrD than to ZSM35 or small port tetra methyl ammonium (TMA) ferrierite.

TABLE 2

X-Ray Comparisons

| Theoretical from Vaughans structure | | Natural Ferrierite Kamploopa | | FU9 Example 1 | | ZSM 35 Table 1 US 4,016,245 | | Zeolite SrD | |
|---|---|---|---|---|---|---|---|---|---|
| h k l | dA | dA | $100^{I}/I_o$ | dA | $100^{I}/I_o$ | dA | R.I. | dA | R.I. |
| 100 | 11.4 | 11.3 | 20 | 11.3 | 7 | – | – | – | – |
| 200 | 9.58 | 9.61 | 100 | 9.50 | 100 | 9.6 | vvs | 9.51 | ms |
| 020 | 7.06 | 7.00 | 30 | 7.05 | 21 | 7.10 | medium | 7.09 | vw |
| 101 | 6.98 | – | – | 6.99 | 22 | 6.98 | medium | 6.96 | vvw |
| 011 | 6.62 | 6.61 | 20 | 6.61 | 19 | 6.64 | medium | 6.63 | ms |
| 310 | 5.82 | 5.84 | 50 | 5.77 | 13 | 5.78 | weak | – | – |
| 220 | 5.69 | – | – | 5.67 | 3 | 5.69 | weak | – | – |
| 121 | 4.964 | 4.96 | 10 | 4.97 | 8 | 4.97 | weak | 4.97 | vw |
| 301 | 4.860 | 4.80 | 10 | 4.84 | 1 | – | – | – | – |
| 400 | 4.790 | – | – | 4.75 | 2 | – | – | 4.75 | w |
| 130 | 4.574 | 4.58 | 10 | 4.57 | 2 | 4.58 | weak | 4.65 | vw |
| 031 | 4.004 | 3.99 | 90 | 3.99 | 53 | 3.99 | strong | – | – |
| 420 | 3.940 | – | – | 3.94 | 37 | 3.94 | m.strong | 3.94 | mw |
| 411 | 3.880 | 3.88 | 10 | 3.85 | 18 | 3.85 | medium | 3.86 | w |
| 330 (002) | 3.791 | 3.79 | 20 | 3.78 | 32 | 3.78 | strong | 3.78 | m |
| 510 (231) | 3.698 | 3.69 | 50 | 3.66 | 14 | 3.74 | weak | 3.73 | vw |
| 112 | 3.557 | – | – | 3.56 | 40 | – | – | – | – |
| 040 | 3.532 | 3.54 | 80 | 3.53 | 55 | 3.54 | v.strong | 3.54 | s |
| 202 | 3.488 | 3.49 | 80 | 3.49 | 52 | 3.49 | v.strong | 3.48 | s |
| 501 | 3.411 | 3.42 | 20 | 3.38 | 10 | 3.39 | weak | 3.39 | vvw |
| 240 | 3.314 | 3.31 | 20 | 3.31 | 15 | 3.32 | weak/ medium | 3.31 | w |
| 141 (312) | 3.152 | 3.15 | 30 | 3.14 | 21 | 3.14 | weak/ medium | 3.14 | ms |
| 521 | 3.072 | 3.07 | 30 | 3.05 | 8 | – | – | 3.06 | ms |
| 530 (402) | 2.972 | 2.97 | 30 | 2.950 | 9 | – | – | 2.96 | w |
| 620 | 2.910 | 2.90 | 20 | 2.898 | 5 | 2.90 | weak | 2.89 | m |
| 422 | 2.722 | 2.72 | 20 | 2.713 | 3 | 2.71 | weak | 2.712 | mw |
| 350 | 2.644 | – | – | 2.643 | 4 | 2.65 | weak | 2.644 | mw |
| 701 | 2.571 | 2.58 | 30 | 2.575 | 3 | 2.58 | weak | 2.580 | vw |

orthorhombic cell

$a_o$ = 19.16
$b_o$ = 14.13
$c_o$ = 7.49

orthorhombic cell

$a_o$ = 19.0
$b_o$ = 14.1
$c_o$ = 7.5

TABLE 3 – SORPTION AT 25°C

| Zeolite | Source | $\frac{SiO_2}{Al_2O_3}$ | $\frac{Na}{Al}$ | Sorption % w/w $P/_{po}$ = 0.5 | | | |
|---|---|---|---|---|---|---|---|
| | | | | n-hexane | cyclohexane | p-xylene | m-xylene |
| FU9 | sodium hydrogen FU9 example 4 this appln. | 20 | 0.21 | 6.2 | <0.2 | 3.3 | 2.5 |
| ZSM 35 | as made per example 1 US 4,016,245 | 28 | 0.22 | 8.5 | 2.1 | 8.0 | 7.7 |
| SrD | H zeolite J Colls Int Sci (1969) *30*, 111 | 12.3 | | 8.8 | <0.2 | 1.5 | 1.2 |
| TMA small port ferrierite | as example 12 this application | 22 | 0.20 | 8.8 | <0.2 | <0.2 | <0.2 |

TABLE 4 – VOIDAGE AVAILABLE

| Zeolite | voidage in cc/100g assuming adsorbed as liquid at 25°C | | | | | |
|---|---|---|---|---|---|---|
| | water | methanol | n-hexane | cyclohexane | p-xylene | m-xylene |
| FU9 | 9.1 | 13.8 | 9.4 | <0.3 | 3.8 | 2.9 |
| ZSM 35 | 9.5 | 13.8 | 12.9 | 2.7 | 9.2 | 8.9 |
| SrD | 9.0 | 14.2 | 13.3 | <0.2 | 1.7 | 1.4 |
| TMA small port | 9.5 | 14.0 | <0.4 | <0.5 | <0.3 | <0.3 |

A further difference between FU9 and ZSM35 can be seen from the Examples by reference to US Patent 4,016,245. FU9 can only be prepared with tetramethyl ammonium compounds, optionally with trialkylamines which may be substituted or their salts, or from tetramethylammonium plus degradation products of tetramethylammonium which can include trimethylhydrogen ammonium and trimethylamine. On the other hand ZSM35 can only be prepared from either ethylene diamine or pyrrolidine. While FU9 can only be prepared with $SiO_2/Al_2O_3$ product ratios from 15 to 30, ZSM35 can be prepared with $SiO_2/Al_2O_3$ ratios from 8 to 50. Attempts to produce FU9 with $SiO_2/Al_2O_3$ less than 15 result in the production of zeolite FU1 (FR—A—2369891) and attempts to synthesise FU9 with $SiO_2/Al_2O_3 > 30$ give rise to FU9 of $SiO_2/Al_2O_3 < 30$ and substantial quantities of α-quartz. Attempts to produce FU9 using only tetramethylammonium can give rise to the production of either FU1 or small port TMA ferrierite.

Finally there is a significant difference in the infra-red spectra of FU9 and ZSM35. FU9 has a single broad peak at 450 $cm^{-1}$, whereas ZSM35 has very well defined double peaks at 465 and 440 $cm^{-1}$.

The invention provides also a method of making zeolite FU9 which comprises reacting an aqueous mixture comprising at least one oxide $XO_2$, at least one oxide $Y_2O_3$ and at least one tetramethylammonium compound wherein the reaction mixture has the molar composition:

| | | |
|---|---|---|
| $XO_2/Y_2O_3$ | 5 to 50 | preferably 10 to 30 |
| free $MO_2/XO_2$ | 0.1 to 1.0 | preferably 0.1 to 0.5 |
| $Z^-/Y_2O_3$ | 0 to 5000 | preferably 10 to 100 |
| $Q/Y_2O_3$ | 0.1 to 150 | preferably 1 to 50 |
| $H_2O/XO_2$ | 5 to 200 | preferably 10 to 30 |
| $Q = (TMA)_2 + xA$ | | |

where X is silicon and/or germanium, Y is one or more of aluminium, gallium, iron, chromium, vanadium, molybdenum, arsenic, manganese or boron, M is an alkali metal or ammonium, and Q is a mixture of TMA the tetramethylammonium compound, amine degradation product thereof or a precursor thereof, or a related compound, and A which is a trialkylamine and/or an alkanolamine or salts thereof, where x is equal to 0.2 to 2.0 moles and A preferably contains 1 to 12 carbon atoms. $Z^-$ is a strong acid radical present as a salt of M and may be added as a free acid to reduce the free $M_2O$ level to a desired value. M and/or Q can be present as hydroxides or salts of inorganic acids provided the $M_2O/XO_2$ requirement is fulfilled.

The preferred quaternary compound is a tetramethylammonium hydroxide.

The preferred alkylamines are trimethylamine, triethylamine or tributylamine.

The preferred alkali metal (M) is sodium. The preferred oxide $XO_2$ is silica ($SiO_2$) and the preferred oxide $Y_2O_3$ is alumina ($Al_2O_3$).

The silica source can be any of those commonly considered for use in synthesising zeolites, for example powdered solid silica. Among the powdered silicas usable are precipitated silicas, especially those made by precipitation from an alkali metal silicate solution, such as the type known as "KS 300" made by AKZO, and similar products, aerosil silicas, fume silicas and silica gels suitably in grades for use in reinforcing pigments for rubber or silicone rubber. Colloidal silicas of various particle sizes may be used, for example 10—15 or 40—50 microns, as sold under the Registered Trade Marks "LUDOX", "NALCOAG" and "SYTON". The usable dissolved silicas include commercially available waterglass silicates containing 0.5 to 6.0, especially 2.0 to 4.0 mols of $SiO_2$ per mol of alkali metal oxide, "active" alkali metal silicates as defined in UK Patent 1193254, and silicates made by dissolving silica in an alkali metal hydroxide or a quaternary ammonium hydroxide or a mixture thereof.

The alumina source is most conveniently sodium aluminate, but can be or can include aluminium, an aluminium salt for example the chloride, nitrate or sulphate, an aluminium alkoxide or alumina itself, which should preferably be in a hydrated or hydratable form such as colloidal alumina, pseudoboehmite, boehmite, gamma alumina or the alpha or beta trihydrate.

The reaction mixture is reacted usually under autogenous pressure, optionally with added gas, e.g. nitrogen at a temperature between 85 and 250°C until crystals of zeolite FU9 form, which can be from 1 hour to many months depending on the reactant composition and the operating temperature. Agitation is optional, but is preferable since it reduces the reaction time.

At the end of the reaction, the solid phase is collected on a filter and washed and is then ready for further steps such as drying.

The invention is illustrated by the following Examples.

Example 1

Preparation of FU9.

The synthesis mixture had the following molar composition:

$$4.5\ Na_2O,\ 3\ QCl,\ Al_2O_3,\ 25\ SiO_2,\ 900\ H_2O$$

where Q = 90% (tetramethylammonium chloride) + 10% trimethylamine, hydrochloride (on a molar basis). First 76.1 g AKZO KS 300 silica ($Na_2O$, 0.187 $Al_2O_3$, 181 $SiO_2$, 54.3 $H_2O$) were suspended in 11.6 g sodium hydroxide dissolved in 700 g water to give slurry A.

Next 15.2 g Q were dissolved in 100 g water to give solution B.

Finally 8.1 g sodium aluminate were dissolved in 41 g water to give solution C.

Then slurry A was heated with stirring to dissolve most of the silica, next solution B was stirrd in followed by Solution C. The mixture was reacted for 24 hours at 180°C in a stirred stainless steel autoclave. After cooling to about 60°C the slurry was filtered and washed with 2 litres of distilled water at about 60°C and then dried overnight at 120°C. The product was sodium organo-FU9 zeolite having typical X-ray diffraction data as shown in Table 1, and having the following composition:

$$0.21\ Na_2O,\ 1.6Q,\ Al_2O_3,\ 21\ SiO_2,\ 5.3\ H_2O$$

The carbon/nitrogen ratio in this product was 3.6 A characteristic of FU9 zeolites as made is that the carbon/nitrogen ratio is always in the range 3 to 3.8. Small port TMA ferrierites typified by the product of Example 10 have carbon/nitrogen ratios of from 4.1 to 4.4.

Example 2

In this example, Q was 100% tetramethyl ammonium chloride. The reaction was carried out as before except that samples were taken at 6 hours, 24 hours and 36 hours respectively.

The product at 6 hours was zeolite FU1, but at 24 hours, 20% of the FU1 had been converted to zeolite FU9. After 36 hours the product was zeolite FU9. These results demonstrate that the degradation products of tetramethylammonium lead ultimately to the production of FU9 even if the first product is zeolite FU1.

Example 3

In this example, the reaction mixture was as in Example 1 and was heated at 230°C for 6 hours. The product was again FU9.

Example 4

In this example, the composition differed from Example 1 in that 100% trimethylamine hydrochloride was used. The product of this reaction was zeolite ZSM8.

Example 5

This example was in Example 1 except that instead of 10% trimethylamine hydrochloride, 7% tributylamine was used. The product was again FU9 of composition:

$$0.1\ Na_2O,\ 2.5\ Q,\ Al_2O_3\ 23.5\ SiO_2,\ 7\ H_2O$$

Example 6

In this example, Q comprised 93% tetramethylammonium chloride and 7% triethanolamine and the mixture ratios were as Example 1. The product was again FU9 of composition:

$$0.19\ Na_2O,\ 1.8Q,\ Al_2O_3,\ 22\ SiO_2,\ 7\ H_2O$$

Example 7

In this example, Q comprised 50% tetramethylammonium chloride and 50% trimethylamine hydrochloride. The mix was otherwise as Example 1 and the product was again FU9.

Example 8

In this modification of Example 1, potassium hydroxide and potassium aluminate replaced the respective sodium compounds. The reaction was 9 hours at 230°C and the product was potassium organo FU9 with X-ray data very similar to the sodium form, differing only marginally in peak positions and peak heights. Its composition was 0.05 $Na_2O$, 0.5 $K_2O$, 1.6 Q, $Al_2O_3$, 17.5 $SiO_2$, 8 $H_2O$.

Example 9

This example gives an unsuccessful attempt to synthesise FU9 having a product ratio $SiO_2/Al_2O_3 > 30$. The molar composition of the reaction mixture was

$$7.2\ Na_2O,\ 4.4\ TMACl,\ 0.4\ \text{trimethylamine HCl},\ 40\ SiO_2,\ 144\ H_2O.$$

The reactants employed were as in Example 1, the reaction ran for 17 hours at 225°C with intermediate sampling. After 6 and 15 hours the products were amorphous (major proportion) and a quartz (minor). After 17 hours the product was major α quartz and minor zeolite FU9.

Example 10

This example illustrates the point that FU9 cannot be synthesised with $SiO_2/Al_2O_3 > 30$, but that it is possible to obtain a near tetramethylammonium ferrierite which has X-ray data much the same as zeolite SrD. This zeolite had small port properties and adsorbed 10.8% w water but less than 0.4% w/w n-hexane.

The composition of the raction mixture was 9 $Na_2O$, 10.8 QCl, $Al_2O_3$, 59.3 $SiO_2$, 26 NaCl, 2000 $H_2O$ Q was as in Example 1.

The reaction after 72 hours at 250°C gave SrD zeolite of composition:

$$0.3\ Na_2O,\ 2.5Q,\ Al_2O_3,\ 47\ SiO_2,\ 16H_2O$$

and the carbon/nitrogen ratio was 4.3.

Example 11

This example illustrates the fact that if insufficient organic base is employed in FU9 type reactions, then the result can be small port ferrierite. The mixture was as follows with Q as in Example 1.

$$4\ Na_2O,\ QCl,\ Al_2O_3,\ 25\ SiO_2,\ 900\ H_2O$$

The product after 9 hours at 230°C was small port ferrierite as in Example 10.

Example 12

The reaction mixture composition was

$$4.2\ Na_2O,\ 3\ QCl,\ Al_2O_3,\ 18\ SiO_2,\ 900\ H_2O$$

Q was as in Example 1. The reaction was for 72 hours at 180°C. The product was FU9 of composition

$$0.3\ Na_2O,\ 1.5Q,\ Al_2O_3,\ 14.8\ SiO_2,\ 4\ H_2O$$

Example 13

The reaction mixture composition was

$$6.8\ Na_2O,\ 4.8\ QCl,\ Al_2O_3,\ 40\ SiO_2,\ 1440\ H_2O$$

Q was as in Example 1. The reaction was for 24 hours at 180°C, the product was FU9 of composition

$$0.5\ Na_2O,\ 1.9Q,\ Al_2O_3,\ 29\ SiO_2,\ 9H_2O$$

**Claims**

1. A synthetic zeolite material, zeolite FU9, as freshly made having a molar composition expressed by the formula:

$$0\ \text{to}\ 0.8\ M_2{:}0.1\ \text{to}\ 20\ Q{:}Y_2O_3{:}10\ \text{to}\ 30\ XO_2{:}0\ \text{to}\ 600\ H_2O$$

wherein M is an alkali metal or ammonium, Q is a tetramethylammonium ion and $M_2O+Q$ is equal to or greater than 1.0, X is silicon and/or germanium, Y is one or more of aluminium, iron, chromium, vanadium, molybdenum, arsenic, manganese, gallium or boron, and $H_2O$ is water of hydration, and having an X-ray diffraction pattern

| | | | | | | |
|---|---|---|---|---|---|---|
| dA | 11,3 | 9,5 | 7,05 | 6,99 | 6,61 | 5,77 |
| $100^I/Io$ | 7 | 100 | 21 | 22 | 19 | 13 |
| dA | 5,67 | 4,97 | 4,84 | 4,75 | 4,57 | 3,99 |
| $100^I/Io$ | 3 | 8 | 1 | 2 | 2 | 52 |
| dA | 3,94 | 3,85 | 3,78 | 3,66 | 3,56 | 3,53 |
| $100^I/Io$ | 37 | 18 | 32 | 14 | 40 | 55 |
| dA | 3,49 | 3,38 | 3,31 | 3,14 | 3,05 | 2,950 |
| $100^I/Io$ | 52 | 10 | 15 | 21 | 8 | 9 |
| dA | 2,898 | 2,713 | 2,643 | 2,617 | 2,575 | 2,545 |
| $100^I/Io$ | 5 | 3 | 4 | 1 | 3 | 1 |
| dA | 2,477 | 2,414 | 2,347 | 2,308 | 2,260 | 2,150 |
| $100^I/Io$ | 3 | 2 | 3 | 1 | 1 | 2 |
| dA | 2,109 | 2,027 | 1,998 | | | |
| $100^I/Io$ | 2 | 2 | 6 | | | |

2. A method of making a synthetic zeolite material, zeolite FU9, which comprises reacting an aqueous mixture comprising at least one oxide $XO_2$, at least one oxide $Y_2O_3$ and at elast one tetramethylammonium compound wherein the aqueous mixture has the molar composition:

| | |
|---|---|
| $XO_2/Y_2O_3$ | 5 to 50 |
| free $MO_2/XO_2$ | 0.1 to 1.0 |
| $Z^-/Y_2O_3$ | 0 to 5000 |
| $Q/Y_2O_3$ | 0.1 to 150 |
| $H_2O/XO_2$ | 5 to 200 |

wherein X, Y and M have the meanings given in claim 1, $Z^-$ is a strong acid radical and

$$Q = (TMA)_2 + xA$$

wherein TMA is a tetramethylammonium compound, A is a trialkylamine or alkanolamine or salt thereof and x is 0.2 to 2.0 moles.

3. A method according to claim 2 wherein $XO_2/Y_2O_3$ is in the range 10 to 30.

4. A method according to claim 2 or 3 wherein free $MO_2/XO_2$ is in the range 0.1 to 0.5.

5. A method according to any one of claims 2 to 4 wherein $Z^-/Y_2O_3$ is in the range 10 to 100.

6. A method according to any one of claims 2 to 5 wherein $Q/Y_2O_3$ is in the range 1 to 50.

7. A method according to any one of claims 2 to 6 wherein $H_2O/XO_2$ is in the range 10 to 30.

8. A method according to any one of claims 2 to 7 wherein the tetramethylammonium compound is tetramethylammonium hydroxide.

**Patentansprüche**

1. Synthetisches Zeolithmaterial, Zeolith FU9, das, wenn es frisch hergestellt ist, eine molare Zusammensetzung hat, die durch die Formel:

$$0 \text{ bis } 0{,}8\ M_2{:}0{,}1 \text{ bis } 20\ Q{:}Y_2O_3{:}10 \text{ bis } 30\ XO_2{:}0 \text{ bis } 600\ H_2O$$

ausgedrückt wird, worin M ein Alkalimetall oder Ammonium ist, Q ein Tetramethylammoniumion ist und

$M_2O+Q$ gleich oder größer als 1,0 ist, X Silicium und/oder Germanium ist, Y ein oder mehr als ein Vertreter von Aluminium, Eisen, Chrom, Vanadium, Molybdän, Arsen, Mangan, Gallium oder Bor ist und $H_2O$ Hydratwasser ist, und das folgende Röntgenbeugungsbild liefert:

| | | | | | | |
|---|---|---|---|---|---|---|
| dA | 11,3 | 9,5 | 7,05 | 6,99 | 6,61 | 5,77 |
| $100^I/Io$ | 7 | 100 | 21 | 22 | 19 | 13 |
| dA | 5,67 | 4,97 | 4,84 | 4,75 | 4,57 | 3,99 |
| $100^I/Io$ | 3 | 8 | 1 | 2 | 2 | 52 |
| dA | 3,94 | 3,85 | 3,78 | 3,66 | 3,56 | 3,53 |
| $100^I/Io$ | 37 | 18 | 32 | 14 | 40 | 55 |
| dA | 3,49 | 3,38 | 3,31 | 3,14 | 3,05 | 2,950 |
| $100^I/Io$ | 52 | 10 | 15 | 21 | 8 | 9 |
| dA | 2,898 | 2,713 | 2,643 | 2,617 | 2,575 | 2,545 |
| $100^I/Io$ | 5 | 3 | 4 | 1 | 3 | 1 |
| dA | 2,477 | 2,414 | 2,347 | 2,308 | 2,260 | 2,150 |
| $100^I/Io$ | 3 | 2 | 3 | 1 | 1 | 2 |
| dA | 2,109 | 2,027 | 1,998 | | | |
| $100^I/Io$ | 2 | 2 | 6 | | | |

2. Verfahren zur Herstellung eines synthetischen Zeolithmaterials, des Zeoliths FU9, bei dem eine wäßrige Mischung, die mindestens ein Oxid $XO_2$, mindestens ein Oxid $Y_2O_3$ und mindestens eine Tetramethylammoniumverbindung enthält, zur Reaktion gebracht wird, wobei die wäßrige Mischung die molare Zusammensetzung:

| | |
|---|---|
| $XO_2/Y_2O_3$ | 5 bis 50 |
| freies $MO_2/XO_2$ | 0,1 bis 1,0 |
| $Z^-/Y_2O_3$ | 0 bis 5000 |
| $Q/Y_2O_3$ | 0,1 bis 150 |
| $H_2O/XO_2$ | 5 bis 200 |

hat, worin X, Y und M die in Anspruch 1 angegebenen Bedeutungen haben, $Z^-$ ein Rest einer starken Säure ist und

$$Q = (TMA)_2 + xA$$

worin TMA eine Tetramethylammoniumverbindung ist, A ein Trialkylamin oder Alkanolamin oder ein Salz davon ist und x 0,2 bis 2,0 mol bedeutet.

3. Verfahren nach Anspruch 2, bei dem $XO_2/Y_2O_3$ in dem Bereich von 10 bis 30 liegt.

4. Verfahren nach Anspruch 2 oder 3, bei dem freies $MO_2/XO_2$ in dem Bereich von 0,1 bis 0,5 liegt.

5. Verfahren nach einem der Ansprüche 2 bis 4, bei dem $Z^-/Y_2O$ in dem Bereich von 10 bis 100 liegt.

6. Verfahren nach einem der Ansprüche 2 bis 5, bei dem $Q/Y_2O_3$ in dem Bereich von 1 bis 50 liegt.

7. Verfahren nach einem der Ansprüche 2 bis 6, bei dem $H_2O/XO_2$ in dem Bereich von 10 bis 30 liegt.

8. Verfahren nach einem der Ansprüche 2 bis 7, bei dem die Tetramethylammoniumverbindung Tetramethylammoniumhydroxid ist.

**Revendications**

1. Matière zéolitique synthétique, appelée zéolite FU9, ayant à l'état fraîchement préparé une composition molaire exprimée par la formule:

$$0 \text{ à } 0{,}8\ M_2{:}0{,}1 \text{ à } 20\ Q{:}Y_2O_3{:}10 \text{ à } 30\ XO_2{:}0 \text{ à } 600\ H_2O$$

dans laquelle M est un métal alcalin ou l'ion ammonium, Q est un ion tétraméthylammonium et $M_2O + Q$ est égal ou supérieur à 1,0 X est le silicium et/ou le germanium, Y est un ou plusieurs des éléments aluminium, fer, chrome, vanadium, molybdène, arsenic, manganèse, gallium et bore, et $H_2O$ représente l'eau d'hydratation, et présentant le diagramme de diffraction des rayons X suivant:

| | | | | | | |
|---|---|---|---|---|---|---|
| dA | 11,3 | 9,5 | 7,05 | 6,99 | 6,61 | 5,77 |
| $100^{I}/Io$ | 7 | 100 | 21 | 22 | 19 | 13 |
| dA | 5,67 | 4,97 | 4,84 | 4,75 | 4,57 | 3,99 |
| $100^{I}/Io$ | 3 | 8 | 1 | 2 | 2 | 52 |
| dA | 3,94 | 3,85 | 3,78 | 3,66 | 3,56 | 3,53 |
| $100^{I}/Io$ | 37 | 18 | 32 | 14 | 40 | 55 |
| dA | 3,49 | 3,38 | 3,31 | 3,14 | 3,05 | 2,950 |
| $100^{I}/Io$ | 52 | 10 | 15 | 21 | 8 | 9 |
| dA | 2,898 | 2,713 | 2,643 | 2,617 | 2,575 | 2,545 |
| $100^{I}/Io$ | 5 | 3 | 4 | 1 | 3 | 1 |
| dA | 2,477 | 2,414 | 2,347 | 2,308 | 2,260 | 2,150 |
| $100^{I}/Io$ | 3 | 2 | 3 | 1 | 1 | 2 |
| dA | 2,109 | 2,027 | 1,998 | | | |
| $100^{I}/Io$ | 2 | 2 | 6 | | | |

2. Procédé de préparation d'une matière zéolitique synthétique, appelée zéolite FU9, qui consiste à faire réagir un mélange aqueux comprenant au moins un oxyde $X_2$, au moins un oxyde $Y_2O_3$ et au moins un composé de tétraméthylammonium, le mélange aqueux répondant à la composition molaire:

| | |
|---|---|
| $XO_2/Y_2O_3$ | 5 à 50 |
| $MO_2$ libre/$XO_2$ | 0,1 à 1,0 |
| $Z^-/Y_2O_3$ | 0 à 5000 |
| $Q/Y_2O_3$ | 0,1 à 150 |
| $H_2O/XO_2$ | 5 à 200 |

où X, Y et M ont les définitions données dans la revendication 1, $Z^-$ est un radical d'acide forte et

$$Q = (TMA)_2 + xA$$

où TMA est un composé de tétraméthylammonium, A est une trialkylamine ou une alcanolamine ou un sel de cette amine et x représente 0,2 à 2,0 moles.

3. Procédé suivant la revendication 2, dans lequel $XO_2/Y_2O_3$ est compris dans l'intervalle de 10 à 30.

4. Procédé suivant la revendication 2 ou 3, dans lequel $MO_2$ libre/$XO_2$ est compris dans l'intervalle de 0,1 à 0,5.

5. Procédé suivant l'une quelconque des revendications 2 à 4, dans lequel $Z^-/Y_2O_3$ est compris dans l'intervalle de 10 à 100.

6. Procédé suivant l'une quelconque des revendications 2 à 5, dans lequel $Q/Y_2O_3$ est compris dans l'intervalle de 1 à 50.

7. Procédé suivant l'une quelconque des revendications 2 à 6, dans lequel $H_2O/XO_2$ est compris dans l'intervalle de 10 à 30.

8. Procédé suivant l'une quelconque des revendications 2 à 7, dans lequel le composé de tétraméthylammonium est l'hydroxyde de tétraméthylammonium.